# EUROPEAN PATENT SPECIFICATION

(11) **EP 2 736 593 B1**
(45) Date of publication and mention of the grant of the patent: **09.09.2015**
(21) Application number: 12735306.8
(22) Date of filing: 16.07.2012
(51) Int. Cl.: A61N 5/06, A61B 18/20

(54) **HANDHOLDABLE LASER DEVICE FEATURING FLEXIBLE CONNECTION BETWEEN A LASER AND A PRINTED CIRCUIT BOARD**
HANDLASERGERÄT MIT FLEXIBLER VERBINDUNG ZWISCHEN EINEM LASER UND EINER LEITERPLATTE
DISPOSITIF LASER PORTABLE AYANT UNE LIAISON FLEXIBLE ENTRE UN LASER ET UNE CARTE DE CIRCUITS IMPRIMÉS

(30) Priority: 28.07.2011 US 201161512430 P
(43) Date of publication of application: 04.06.2014
(73) Proprietor: ILUMINAGE BEAUTY LTD, P.O.B. 14 (IL)
(72) Inventor: PARENT, Thomas, Gerard, Cambridge, Massachusetts 02138 (US)
(74) Representative: ZBM Patents - Zea, Barlocci & Markvardsen
(86) International application number: PCT/EP2012/063920
(87) International publication number: WO 2013/014016

(56) References cited:
- WO-A1-2010/079834
- US-A- 4 595 838
- US-A- 5 150 704

## Description

### BACKGROUND OF THE INVENTION

### Field of the Invention

The invention concerns a cordless handholdable laser device with internal circuitry allowing angular orientation of output beam and size reduction. The laser device is useful to treat wrinkles and hyperpigmentation.

### The Related Art

Devices based on light amplification by stimulated emission of radiation (laser) have revolutionized many areas of dermatological medicine and of cosmetics. Amongst skin conditions responsive to treatment are acne scars, rosacea, hyperpigmentation, unwanted hair and dermal rejuvenation. Ablative resurfacing has become a common method for cosmetic rejuvenation. Wrinkle reduction has been a particular objective of the phototherapy.

Advances in laser based devices and their use in skin treatment methods have been many during the last decade. Several publications have focused on safe arming of the device to avoid unintended exposures. US 2004/0167502 A1 (Weckwerth et al.) reports optical sensors for detecting engagement with a skin surface. The sensors are based upon multiple light emitting diodes, each having a unique wavelength band, and a broad-band photodetector to measure the remission of light at multiple wavelengths from a material being analyzed. US 2010/0082020 (Gong et al.) describes a medical laser having a capacitance sensor and an emission control device to ensure that a laser handpiece is in contact with skin prior to activation. The handpiece needs to stand perpendicular to the skin surface before any surgical operation begins.

Most electromagnetic radiation delivery devices for treatment of skin are relatively large pieces of equipment. Complexity in their basic engineering and mode of operation defeats miniaturization into a handheld device. For instance, US 2008/0082089 A1 (Jones et al.) describes a system including a first solid-state and a second solid-state laser. A respective first output beam is fed into the second device for generating excitation in a rare earth doped gain medium to produce a second output beam. The latter is used to treat skin. US 2007/0179481 A1 (Frangineas et al.) seeks to treat skin laxity with a plurality of pulses from a carbon dioxide laser. The system requires a housing to contain a scanning apparatus and a tip connected to a vacuum pump for exhausting smoke resulting from ablation.

Many of the reported ablative procedures require special cooling mechanisms. For instance, US 5 810 801 directs a beam of radiation to penetrate the dermal region below a wrinkle to injure collagen. A cooling system is then activated to prevent injury of the overlying epidermis. These cooling systems are often quite bulky.

Another problem with the state of the art, particularly with portable instruments, is in their effectiveness to emit sufficiently energetic doses of electromagnetic radiation. US 2011/0040358 A1 (Bean et al.) provides one solution describing a portable device which is eye safe operating between 1350-1600 nm to treat wounds and diseases. This is a battery operated system that need not directly contact tissue. A key part of the device is a lens constructed to have the laser beam converge to a focal point slightly above the tissue surface target.

US 2007/198004 A1 discloses a laser device for treating skin a with a laser member within a hand-holdable housing, a battery and a a flexible electrically conductive connector communicating the laser member with a printed circuit board.

### SUMMARY OF THE INVENTION

A laser device for treating skin is provided which includes:
(i) a handholdable housing;
(ii) a laser member arranged within the housing and emitting an output beam;
(iii) a printed circuit board mounted on a support block within the housing; and
(iv) a flexible electrically conductive connector having forward and rear ends, the forward end electrically communicating with the laser member, the rear end electrically communicating with the printed circuit board, an area between the ends allowing the connector to bend through an angle range of from 0 to 360°.

The invention is as defined in the appended claims.

### BRIEF DESCRIPTION OF THE DRAWING

Further features, aspects and benefits of the present invention will become more readily apparent from consideration of the following drawing in which:
Fig. 1 is a front view of one embodiment of the invention;
Fig. 2 is a plan perspective view of the embodiment according to Fig. 1;
Fig. 3 is a cross sectional view of Fig. 1 taken perpendicular to that view;
Fig. 4 is a view of the internal mechanism separated from the housing of Fig. 1;
Fig. 5 is a semi-schematic view of a portion of Fig. 4 encompassing the laser and printed circuit board; and
Fig. 6 is an electrical overview of circuits for the shown embodiment.

### DETAILED DESCRIPTION OF THE INVENTION

Now we have developed a laser device in a highly compact handholdable configuration for treating skin. In a preferred format, the device is cordless and meets power needs through a rechargeable battery. Most advantageously, the flexible electrically conductive connector arrangement between the printed circuit board and the laser achieves the best geometry of beam angle to housing handle that translates to a safer operation. For instance, a first end of the housing which emits the electromagnetic radiation can be bent 100 to 170° relative to a handle area near the second end of the housing. A user can therefore read power levels and activation signals on the handle simultaneously with pressing the first end against the face.

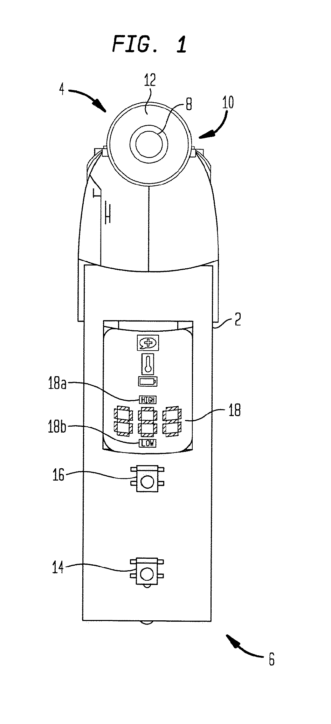

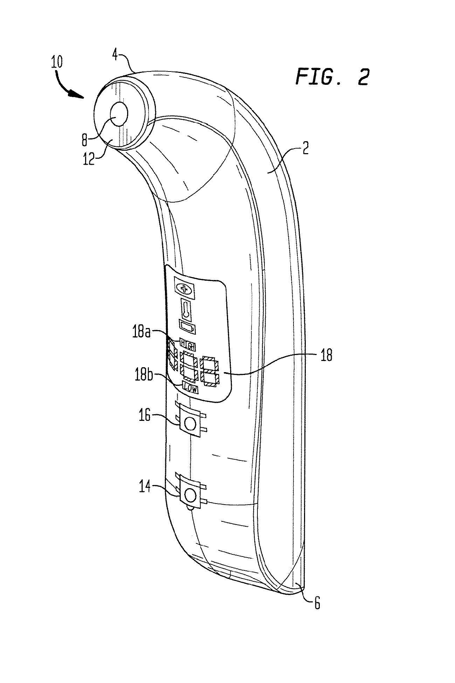

Fig. 1 and 2 reveal a first embodiment of this invention. The laser device features a curvilinear housing 2 having a first end 4 and an opposite second end 6. An aperture defining a window 8 is formed at a tip 10 of the first end of the housing.

The housing preferably has a sinusoidal or S-shape. This allows the tip 10 to be properly oriented against a user's face and simultaneously permits viewing by the user of power settings and activation. A longitudinal axis along a length of the housing and an axis traversing through the window at a point of intersection will define an angle between 100° and 170°, preferably between 110° and 160°, and optimally between 120° and 140°.

An annular plate 12 surrounds window 8. The plate is opaque to electromagnetic radiation. Any output beam of electromagnetic radiation is emitted through the window 8 which is an open central area of the annular plate.

Two control buttons are activatable from outside the housing. One is a power activating button 14 functioning to arm/power on the device. The other is a power setting button 16 functioning to control the power level. The term "button" is to be interpreted broadly. Although in the first embodiment, the buttons are square, these may in other embodiments be of a round or other geometrical shape. Also these buttons may be movable inward/outward from a surface of the housing, but in another embodiment may be a non-movable touch screen form of switch.

In conjunction with the power setting button, there is a light emitting diode (LED) 18 for indicating the setting of high or low power 18a and 18b.

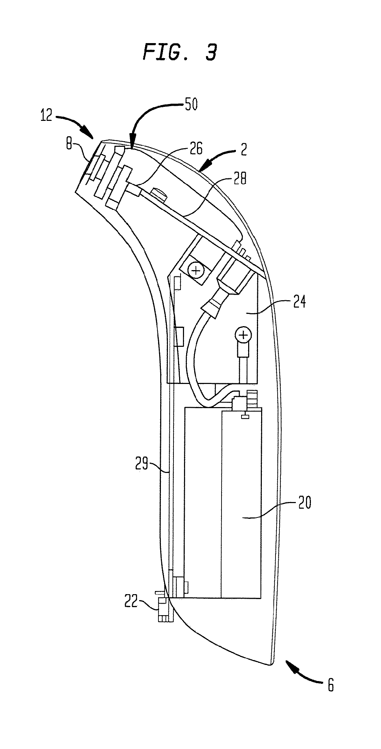

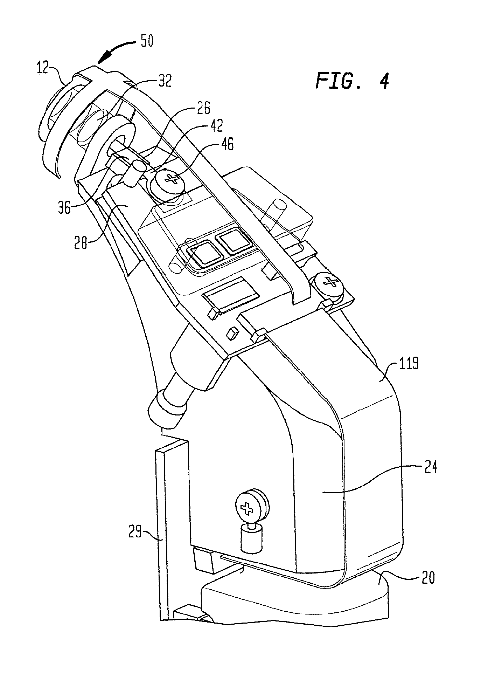

Fig. 3 and 4 reveal the inner mechanism of the laser device. A rechargeable battery 20 is lodged within a lower area of the housing just above the rear end 6. Recharging is achieved by connection of an outside power source to port 22 electrically communicating with the rechargeable battery.

Above the battery is an aluminum block 24 serving both as a support and solid coolant to dissipate heat generated by the laser member. The device neither needs nor features any special liquid or gas coolant system.

A laser member 26 generating electromagnetic radiation is supported on an arm of the aluminum block. The laser member of this embodiment operates on a constant output power delivering a continuous wave over time. It is a solid state diode laser including the elements indium, arsenic, gallium and tin. The laser produces a pulse of radiation having a wavelength between approximately 1300 and 1600 nm, preferably between 1420 and 1470 nm, and optimally about 1440 nm. Fluence may range between 0.5 and 5 joules/cm², more preferably between 1 and 3 joules/cm², and optimally between 1.3 and 1.8 joules/cm². Electromagnetic radiation emanating from the laser device is non-ablative to the skin being classified by the U.S. Food & Drug Administration as a Class I/1 inherently safe rating.

No lotions, creams or other chemicals need be applied to the skin target prior to the radiation treatment. The device of this invention needs no boost nor interacts with pre-positioned chemicals on the skin target area. Nonetheless, it may be desirable to cleanse the skin treatment area with a surfactant composition to avoid interference from makeup or other chemicals that might shield against the efficacy of applied electromagnetic radiation.

Upstream from the laser member 26 is a laser printed circuit board 28 supported on an arm of the aluminum block 24. Operation of the device is controlled by the laser printed circuit board 28 and a main printed circuit board 29. These boards regulate power switching, radiation fire sequencing, generation, timing, sequencing of laser pulses and processing of skin contact information.

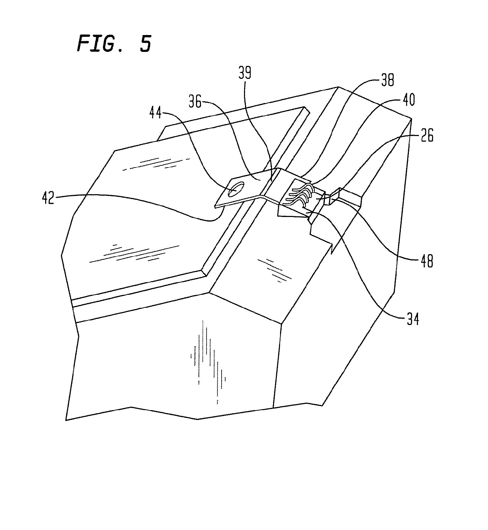

Between the laser member 26 and the aluminum block 24 is a submount 34 as best seen in Fig. 5. The submount directly supports the laser member and also a flexible electrically conductive connector 36 carrying signals/current from the printed circuit board 28. The flexible electrically conductive connector features forward and rear ends 38, 42. An area 39 between the forward and rear ends is highly bendable. The bending may range from 0 to 360° in angle. This allows various angles between a major plane of the laser member and a major plane of the printed circuit board. Preferably, the angle is held between 10 and 250°. This flexibility in orientation creates a geometric and ergonomic advantage.

The forward end of the flexible connector is bonded to the submount. A portion of the forward end features a set of several wire bonds 40 which complete the electrical connection to the laser member 26. The rear end 42 of the flexible connector features an aperture 44 for a screw 46 or other fastening member to achieve a press contact with the printed circuit board. The screw and a washer assembly provides an evenly distributed force which compresses a large area of the flexible connector to a plated contact on the printed circuit board. This arrangement minimizes contact resistance, thus lowering electrical power loss. This arrangement also allows for ease of assembly, disassembly and replacement.

The flexibility of the connector allows the system to escape the ordinarily required connection of circuitry to be in a plane of the output beam generated by the laser. Flexible connectors in one embodiment of this invention are formed of a set of copper wires sandwiched between layers of polyimide.

Fashioned in a downstream area of the submount 34 is an alignment structure 48 with outwardly tapering walls. The alignment structure receives the forward end 38 of the flexible connector to prevent movement and insuring the laser member is properly oriented.

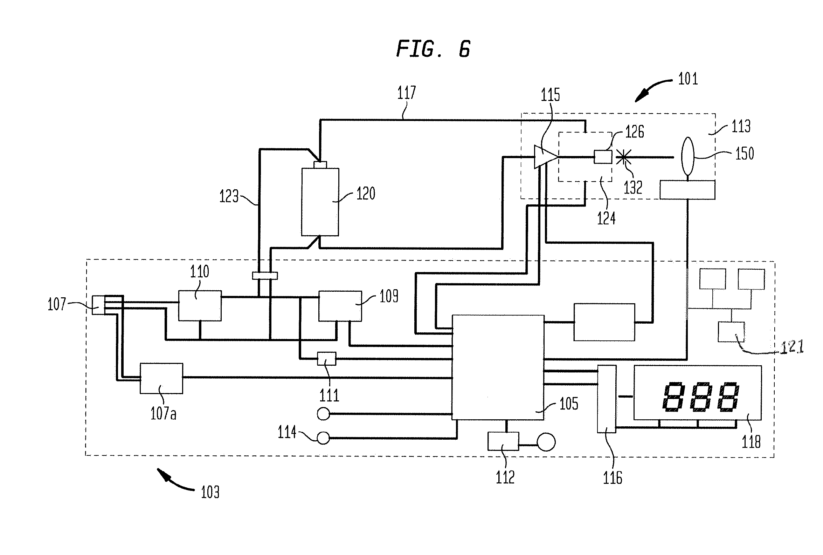

Fig. 6 reveals electrical relationships among elements constituting one embodiment of this invention. A problem with rechargeable battery operated devices is loss of power over time. The device of this invention may have an embodiment which is idled for long periods of time between uses. Consequently, it is necessary to draw as little power as possible from the battery 120 when in the sleep mode. This objective is achieved by having two power domains 101, 103. The first power domain 101 is controlled by a main circuit board 105 which draws very little current (around half a milliamp) when placed into a sleep mode. Wake up occurs when the main circuit board receives a signal generated either by the power button 114 or detection of a USB connectivity 107 via a USB serial communicator 107a. In sleep or shut down mode, voltage regulators 109, 111 which supply 3.3 and 5.0 volt power to the main circuit board are turned off.

Other features of the first power domain include a charger 110 for the battery, audio output 112, driver 116 for LED 118, and a real time clock 121. The real time clock is present for time stamping even when the laser device is shut down. An important feature of the real time clock is enforcement of a 24 hour delay between skin treatments. This mechanism is completely independent of the other safety control mechanisms that shut down operations on the main circuit board, and thereby serves as a double safety precaution against skin over exposure to laser radiation.

The second power domain 103 is controlled by a laser printed circuit board 113. Among components of board 113 is a laser enabling drive 115, laser member 126, aluminum block 124 (functioning as a heat sink), diffractive lens array 132 and contact (capacitive) sensor 150.

The battery 120 has two sets of leads. A first set of leads 117 connects directly to the laser printed circuit board 113 and heat sink 124. The first set of leads provides a high current and low resistance path for the current (around 30 amps) to run the laser member 126. Typically leads 117 may be an 16 gauge wire having DC resistance of 0.013 ohms per meter and an area of 1.3 mm². Lead wire for the first set may range in resistance from 0.004 to 0.06 ohms. Battery current flows from the positive high-current lead through the heat sink and the laser, exiting the cathode of the laser through a flexible circuit then reconnecting to the laser printed circuit board. A ribbon cable 119 connects the laser printed circuit board to the main circuit board.

A second set of leads 123 connect battery 120 to the main circuit board 105. Wires for this connection are much thinner than those used in the first set of leads 117. For instance, leads 117 may be of 24 gauge wire with a DC resistance of 0.086 ohms per meter and a cross sectional area of 0.2 mm². Lead wire for the second set may range in resistance from 0.065 to 0.2 ohms. The second set of leads power control circuitry on the main circuit board and on the laser printed circuit board. The battery is also charged through leads 117.

Use of two separate power connections to the battery avoids having to run any significant amount of power through the ribbon cable 119. This would be necessary were power only to come through the laser printed circuit board. The arrangement eliminates need for extra conductors in the ribbon cable and reduces electrical noise that might arise from extended wiring.

Benefit of having separate high power (laser) and low power (connected to circuit boards) is greater efficiency on space and improved safety because the circuit board must first be activated before the laser can be energized.

Downstream from the laser member arranged near the first end 4 is a diffractive lens array 32. An output beam of electromagnetic radiation from the laser member 26 is directed into the lens array which serves as a prism splitting the output beam into multiple beamlets. These beamlets constitute a larger diameter overall beam exiting the array and possess a non-uniform energy profile. Within the profile are a plurality of high-intensity zones surrounded by lower-intensity zones. Use of the defractive lens array allows the exiting beamlets to strike a broader area of the target skin. The higher-intensity zones heat selected portions of the target skin causing collagen shrinkage while the lower-intensity zones provide sufficient energy to stimulate collagen production. This combination allows a large area of the skin target to be treated simultaneously while minimizing the risk of burning or other damage to the skin.

A capacitive sensor electrode 50 best seen in Fig. 1 and 4 is positioned at the first end 4 of the housing. An electrode terminates on each of three 120° sectors of the annular plate 12. A gap separates each of the sectors. The three electrodes are arranged in an annulus (representing a plane) to determine when a flat surface of suitable dielectric (i.e. skin) is sensed. By arranging the electrodes in a ring, they stay concentric to the cross section of the window 8 through which the electromagnetic radiation is emitted. The arrangement maximizes the surface area of the sensor and allows maintenance of the smallest possible volume around the window 8.

The capacitive sensor includes two conductors with a capacitance field between them. There are three capacitive switches related to each of the three electrodes. Each of the switches must satisfy a condition that it has the capacitance correlated with proper dry skin contact. When there is only partial contact with the skin, the dielectric is improper and firing of the laser cannot occur.

In summary, the present invention is described above in terms of a preferred and other embodiments. The invention is not limited, however, to the described and depicted embodiments. Rather, the invention is only limited by the claims appended hereto.

## Claims

1. A laser device for treating skin comprising:
(i) a handholdable housing (2);
(ii) a laser member (26) arranged within the housing and emitting an output beam; wherein the output beam emits electromagnetic radiation of wavelength ranging from 1420 to 1470 nm; and wherein the output beam has a fluence range from 0.5 to 5 joules/cm²;
(iii) a printed circuit board (28) mounted on a support block (24) within the housing; wherein the support block comprises aluminum;
(iv) a flexible electrically conductive connector (36) having forward (38) and rear ends (42), the forward end electrically communicating with the laser member, the rear end electrically communicating with the printed circuit board and an area between the ends (39) allowing the connector to bend through an angle range of from 0 to 360°; wherein the rear end has an aperture for a screw or other fastener to join the connector with the printed circuit board; and wherein the printed circuit board and the laser member are each defined by major planes, the planes being angled to one another over a range from 10 to 250°; and
(v) a rechargeable battery (20) and two sets of electrical leads (117, 123), each set having a connection to a cathode and an anode of the rechargeable battery.

2. A device according to claim 1 wherein the output beam has a fluence range from 1 to 3 joules/cm².

3. A device according to any one of the preceding claims wherein the angle of bend ranges from 10 to 250°.

4. A device according to any one of the preceding claims wherein the connector is a set of copper wires sandwiched between layers of polyimide.

5. A device according to any one of the preceding claims wherein the forward end has exposed copper wires attached to the laser member.

6. A device according to any one of the preceding claims wherein one of the two sets of electrical leads has a wire gauge smaller than a second of the two sets of electrical leads.

## Patentansprüche

1. Ein Lasergerät zur Behandlung der Haut umfassend:
(i) ein handhaltbares Gehäuse (2);
(ii) ein Laserelement (26), das im Gehäuse angeordnet ist und einen Ausgangsstrahl ausstrahlt; wobei der Ausgangsstrahl elektromagnetische Strahlung einer Wellenlänge reichend von 1420 bis 1470 nm ausstrahlt; und wobei der Ausgangsstrahl einen Fluenzbereich von 0,5 bis 5 joules/cm² hat;
(iii) eine auf einem Stützblock (24) im Gehäuse montierte Leiterplatte (28); wobei der Stützblock Aluminium umfasst;
(iv) ein flexibles elektrisch leitendes Anschlussteil (36), welches vordere (38) und hintere Enden (42) hat, wobei das vordere Ende elektrisch verbunden mit dem Laserelement ist, das hintere Ende elektrisch verbunden mit der Leiterplatte ist und ein Mittelbereich zwischen den Enden (39) eine Biegung des Anschlussteils durch einen Winkelbereich von 0 bis 360° ermöglicht; wobei das hintere Ende eine Öffnung für eine Schraube bzw. ein anderes Befestigungsmittel hat, um das Anschlussteil mit der Leiterplatte zu verbinden; und wobei die Leiterplatte und das Laserelement jeweils durch Hauptebenen definiert sind, wobei die Ebenen miteinander einen Winkel von 10 bis 250° bilden; und
(v) eine wiederaufladbare Batterie (20) und zwei Reihen von elektrischen Leitungen (117, 123), wobei jede Reihe eine Verbindung mit einer Kathode und einer Anode der wiederaufladbaren Batterie hat.

2. Ein Gerät nach Anspruch 1, wobei der Ausgangsstrahl einen Fluenzbereich von 1 bis 3 joules/cm² hat.

3. Ein Gerät nach einem der vorhergehenden Ansprüche, wobei der Biegewinkel von 10 bis 250° reicht.

4. Ein Gerät nach einem der vorhergehenden Ansprüche, wobei das Anschlussteil eine Reihe von zwischen Polyimidschichten eingelegten Kupferdrähten ist.

5. Ein Gerät nach einem der vorhergehenden Ansprüche, wobei das vordere Ende freiliegende Kupferdrähte hat, die an dem Laserelement befestigt sind.

6. Ein Gerät nach einem der vorhergehenden Ansprüche, wobei eine der zwei Reihen von elektrischen Leitungen einen Leitungsquerschnitt hat, der kleiner als derjenige einer zweiten der zwei Reihen von elektrischen Leitungen ist.

## Revendications

1. Un dispositif laser pour le traitement de la peau comprenant :
(i) un boîtier (2) pouvant être tenu à la main ;
(ii) un élément laser (26) disposé dans le boîtier et émettant un faisceau de sortie ; dans lequel le faisceau de sortie émet du rayonnement électromagnétique d'une longueur d'onde allant de 1420 à 1470 nm; et dans lequel le faisceau de sortie a une gamme de fluence de 0,5 à 5 joules/cm²;
(iii) un circuit imprimé (28) monté sur un bloc du soutien (24) dans le boitier ; dans lequel le bloc du soutien comprend de l'aluminium ;
(iv) un connecteur électroconducteur flexible (36) ayant des extrémités avant (38) et arrière (42), communicant l'extrémité avant éléctriquement avec l'élément laser, l'extrémité arrière communicant éléctriquement avec le circuit imprimé et une région située entre les extrémités (39) permettant la flexion du connecteur sur une plage angulaire de 0 à 360° ; dans lequel l'extrémité arrière a une ouverture pour une vis ou pour un autre élément de fermeture afin de relier le connecteur avec le circuit imprimé ; et dans lequel le circuit imprimé et l'élément laser sont chacun définis par des plans principaux, étant les plans disposés l'un par rapport l'autre à un angle allant de 10 à 250 ° ;
(v) une batterie rechargeable (20) et deux séries de lignes électriques (117, 123), ayant chaque série une connexion avec une cathode et une anode de la batterie rechargeable.

2. Un dispositif selon la revendication 1 dans lequel le faisceau de sortie a une gamme de fluence de 1 à 3 joules/cm².

3. Un dispositif selon l'une quelconque des revendications précédentes dans lequel l'angle de flexion va de 10 à 250°.

4. Un dispositif selon l'une quelconque des revendications précédentes dans lequel le connecteur est une série de fils de cuivre placés en sandwich entre des couches de polyimide.

5. Un dispositif selon l'une quelconque des revendications précédentes dans lequel l'extrémité avant a des fils de cuivre exposés attachés à l'élément laser.

6. Un dispositif selon l'une quelconque des revendications précédentes dans lequel une des deux séries de lignes électriques a un calibre de fils plus petit qu'une deuxième des deux séries de lignes électriques.
